# EUROPÄISCHE PATENTSCHRIFT

(11) **EP 1 842 547 B1**
(45) Veröffentlichungstag und Bekanntmachung des Hinweises auf die Patenterteilung: **06.01.2010**
(21) Anmeldenummer: 06011779.3
(22) Anmeldetag: 07.06.2006
(51) Int. Cl.: A61K 36/714, A61P 31/12

(54) **Arzneimittel und/oder Nahrungsergänzungsmittel enthaltend Nisylen, Cepa, Euphrasia, Belladonna und/oder Mercurius solubilis**
Pharmaceutical or nutritional supplement comprising nisyline, cepa, euphrasia, belladonna and/or mercurius solubilis
Composition pharmaceutique ou complément alimentaire contenant de la nisyline, de cepa, eurphrasia, belladona et/ou de mercurius solubilis

(30) Priorität: 28.03.2006 DE 202006004962 U; 30.03.2006 EP 06006727
(43) Veröffentlichungstag der Anmeldung: 10.10.2007
(73) Patentinhaber: Huber, Klaus, 83646 Bad Tölz (DE)
(72) Erfinder: Huber, Klaus, 83646 Bad Tölz (DE)
(74) Vertreter: Herrmann, Uwe

(56) Entgegenhaltungen:
- WO-A-2004/035071
- US-B1- 6 641 801
- DATABASE MEDLINE [Online] US NATIONAL LIBRARY OF MEDICINE (NLM), BETHESDA, MD, US; Juli 1955 (1955-07), BUCHSEL H W: "[Report of this year's influenza treated with nisylen.]" XP002417561 Database accession no. NLM13267864 & THERAPIE DER GEGENWART JUL 1955, Bd. 94, Nr. 7, Juli 1955 (1955-07), Seiten 264-265, ISSN: 0040-5965
- DATABASE WPI Week 200557 Derwent Publications Ltd., London, GB; AN 2005-560398 XP002433905 & RU 2 253 472 C1 (GAKKEL T A) 10. Juni 2005 (2005-06-10)

## Beschreibung

Die vorliegende Erfindung betrifft ein Arzneimittel und/oder Nahrungsergänzungsmittel enthaltend Nisylen, Cepa, Euphrasia, Belladonna und/oder Mercurius solubilis sowie die Verwendung von Nisylen und/oder Cepa und/oder Euphrasia und Belladonna und/oder Mercurius solubilis für ein Arzneimittel und/oder Nahrungsergänzungsmittel bzw. für deren Herstellung.

Nisylen ist als Arzneimittel zur Anwendung bei grippalen Infekten, Fieber, Hals- und Kopfschmerzen und Husten bekannt. Es handelt sich um ein homöopathisches Arzneimittel in Form eines Kombinationspräparates aus verschiedenen Wirkstoffen, das in Form von Tabletten oder einer Lösung zum Einnehmen erhältlich ist. Bei den Wirkstoffen handelt es sich um Aconitum Dil. D3, Gelsemium Dil. D3, Ipecacuanha Dil. D3, Phosphorus Dil. D5, Bryonia Dil. D2 und Eupatorium perfoliatum.

Ebenfalls bekannt ist die Verwendung von Cepa, Euphrasia, Belladonna und Mercurius solubilis für homöopathische Arzneimittel.

Aus der Druckschrift "Therapie der Gegenwart, Juli 1955, Seiten 264-265, Buchsel, H.W." ist ferner die Verwendung von Nisylen zur Behandlung von Influenza bekannt.

Die Aufgabe der vorliegenden Erfindung besteht darin, weitere medizinische Verwendungen einer oder mehrerer der oben angegebenen Bestandteile anzugeben.

Die Erfindung basiert auf der Erkenntnis, dass Nisylen und/oder Cepa und/oder Euphrasia und/oder Belladonna und/oder Mercurius solubilis antivirale Wirkungen, insbesondere bei der präventiven und/oder therapeutischen Behandlung von Herpes labialis und/oder Herpes genitalis eingesetzt werden kann/können.

Bei Herpes labialis handelt es sich um eine Viruserkrankung, die durch das Herpes-Simplex-Virus Typ I (HSV Typ I) hervorgerufen wird. Dabei entstehen an den Lippen kleine, nässende Bläschen, die empfindlich sind, Schmerzen und Juckreiz verursachen und hochinfektiös sind.

Herpes genitalis wird durch den Herpes-Simplex-Virus Typ II (HSV Typ II) hervorgerufen. Auch bei Herpes genitalis bilden sich mit Flüssigkeit gefüllte Bläschen, die erhebliche Schmerzen, Neuralgien und Juckreiz verursachen und ebenfalls hochinfektiös sind.

Beide Virusarten (HSV Typ I und II) befallen Haut, Schleimhäute, Augen, das Nervensystem und selten auch innere Organe. Aufgrund der Tatsache, dass sich das Virus ständig im Körper befindet, kann es immer wieder zu Rezidiven, d.h. erneuten Ausbrüchen der Krankheit kommen. Bei den derzeit bekannten Medikamenten handelt es sich um Virustatika, die den Krankheitsverlauf verkürzen und die Beschwerden mindern.

Erfindungsgemäß wurde nun gefunden, dass ein Arzneimittel bzw. Nahrungsergänzungsmittel, das Nisylen und Cepa und Euphrasia und Belladonna und Mercurius solubilis enthält, antivirale Wirkungen zeigt und beispielsweise zur präventiven und therapeutischen Behandlung von Herpes dient.

Die nachstehenden Ausführungen zu dem erfindungsgemäßen Arzneimittel gelten entsprechend für das erfindungsgemäße Nahrungsergänzungsmittel.

Die Behandlung von Patienten mit Herpes labialis sowie von Patienten mit Herpes genitalis mit dem Arzneimittel gemäß der vorliegenden Erfindung hat gezeigt, dass sich der Krankheitsverlauf in beiden Fällen erheblich verkürzt hat.

Erfindungsgemäß kann vorgesehen sein, dass das Arzneimittel einen der Wirkstoffe Nisylen oder Cepa oder Euphrasia oder Belladonna oder Mercurius solubilis als Hauptbestandteil und die weiteren Bestandteile als Nebenbestandteile enthält, wobei der Hauptbestandteil in größerer Menge bzw. Konzentration vorliegt als jeder der weiteren Bestandteile.

Von der Erfindung sind beliebige Kombinationen der genannten Bestandteile umfaßt, beispielsweise ein Arzneimittel das Nisylen als Hauptbestandteil und alle anderen der genannten Bestandteile als Nebenbestandteile umfaßt.

Es ist vorgesehen, dass das Arzneimittel Nisylen enthält.

Erfindungsgemäß ist vorgesehen, dass das Arzneimittel ferner Cepa enthält. Bei Cepa handelt es sich ebenfalls um eine homöopathisch wirksame Substanz, die üblicherweise bei Schnupfen angewendet wird.

Erfindungsgemäß enthält das Arzneimittel ferner Euphrasia. Euphrasia ist ebenfalls eine aus der Homöopathie bekannte Substanz, die üblicherweise bei Erkrankungen der Augen eingesetzt wird.

Weitere Bestandteile des Arzneimittels gemäß der vorliegenden Erfindung sind Belladonna und Mercurius solubilis . Belladonna wird derzeit als homöopathisches Medikament bei verschiedenen Formen von Entzündungen eingesetzt. Mercurius solublilis ist ein homöopathisches Medikament, das zur Behandlung unter anderem von Halsentzündung, Mittelohrentzündung und Heiserkeit Verwendung bekannt ist.

In weiterer Ausgestaltung der Erfindung ist denkbar, dass - sofern in dem Arzneimittel enthalten - die Bestandteile Cepa, Euphrasia und Mercurius solubilis in der Potenz D12 und Belladonna in der Potenz D15 vorliegen. Grundsätzlich ist es ebenfalls denkbar, die Wirkstoffe in davon abweichenden Potenzen einzusetzen.

Es hat sich als besonders vorteilhaft erwiesen, wenn der Bestandteil Nisylen in dem Arzneimittel in größerer Menge bzw. Konzentration vorliegt als die weiteren Bestandteile.

Vorzugsweise enthält das Arzneimittel Nisylen, Cepa, Euphrasia, Belladonna und Mercurius solubilis, wobei Nisylen in dem Arzneimittel in größerer Menge vorliegt als die weiteren genannten Bestandteile.

Besonders vorteilhaft ist es, wenn der Bestandteil Nisylen in zwei- bis vierfacher, vorzugsweise in dreifacher Menge vorliegt, wie jeder der weiteren Wirkstoffe des Arzneimittels.

Nach einer weiteren Ausgestaltung der Erfindung ist vorgesehen, dass das Arzneimittel Nisylen in einem Gewicht oder in einem Volumen von 4,0 bis 4,5 g oder ml, vorzugsweise in einem Gewicht oder einem Volumen von 4,286 g oder ml, und Cepa, Euphrasia, Belladonna und Mercurius solubilis jeweils in einem Gewicht oder in einem Volumen von 1,2 bis 1,6 g oder ml, vorzugsweise in einem Gewicht oder in einem Volumen von 1,429 g oder ml je 10 ml oder auch je 10 g Lösung bzw. Arzneimittel bzw. Nahrungsergänzungsmittel enthält.

Es ist denkbar, das Arzneimittel zum Einnehmen und/oder zum Auftragen auszuführen. Letzteres kann beispielsweise bei der Behandlung von Herpes labialis von Relevanz sein.

In bevorzugter Ausgestaltung liegt das Arzneimittel in flüssiger Form vor, wobei es sich um eine alkoholische Lösung zum Einnehmen handeln kann. Auch andere Lösungsmittel als Alkohol sind denkbar. Grundsätzlich ist ebenfalls denkbar, das Arzneimittel in fester Form, beispielsweise als Pulver, Tablette oder als Globuli vorzusehen. Auch ist es möglich, das Arzneimittel in pastöser Form bzw. als Salbe bereitzustellen.

Bei dem Arzneimittel handelt es sich vorzugsweise um ein homöopathisches Arzneimittel.

Der Bestandteil Nisylen kann folgende Bestandteile enthalten: Aconitum napellus, Gelsemium sempervirens, Cephaelis ipecacuanha, Phosphorus, Bryonia und Eupatorium perfoliatum. Denkbar ist, dass pro 1 g Nisylen die Bestandteile in folgenden Mengen enthalten sind: 100 mg Aconitum napellus D3, 100 mg Gelsemium sempervirens D3, 100 mg Cephaelis ipecacuanha D3, 100 mg Phosphorus D5, 100 mg Bryonia D2 und 100 mg Eupatorium perfoliatum D1.

Weitere Einzelheiten und Vorteile der Erfindung werden anhand eines im Folgenden dargestellten Ausführungsbeispiels näher erläutert.

Das Arzneimittel gemäß dem hier beschriebenen Ausführungsbeispiel enthält folgende Bestandteile:

| | |
|---|---|
| Nisylen: | 4,286 g |
| Cepa D12: | 1,429 g |
| Euphrasia D12: | 1,429 g |
| Belladonna D15: | 1,429 g |
| Mercurius solubilis D12: | 1,429 g. |

Diese Bestandteile liegen einer alkoholischen Lösung in einem Volumen von 10 ml vor.

Wie oben ausgeführt, ist eine derartige Lösung in hervorragender Weise unter anderem zur Behandlung von Herpes genitalis sowie von Herpes labialis geeignet.

Dabei kann vorgesehen sein, dass beispielsweise 3 x 5 Tropfen pro Tag bei Erwachsenen und 3 x 2 Tropfen pro Tag bei Kindern eingenommen werden. Besonders bevorzugt ist eine regelmäßige Einnahme des Arzneimittels.

Denkbar ist ebenfalls, im akuten Stadium oder bevorzugt im Prodromalstadium eine halbstündliche bis stündliche Applikation, das heißt pro halber Stunde bis Stunde z.B. fünf (Erwachsene) bzw. zwei Tropfen (Kinder) der Lösung einzunehmen.

Als vorteilhaft hat es sich erwiesen, die Lösung nicht zu verdünnen und möglichst lange im Mund zu behalten. Als vorteilhaft hat es sich weiter erwiesen, viel zu trinken, möglichst ca. 3 1 / Tag.

Wie oben ausgeführt, kommt es bei den oben genannten Herpes-Erkrankungen häufig zu Rezidiven, die ein- oder mehrmals pro Jahr, ggf. auch monatlich auftreten können. Die bisher bekannten Medikamente helfen im akuten Stadium durch Abkürzung des Krankheitsverlaufes und ggf. Linderung der Beschwerden, können jedoch nicht das Auftreten der Rezidive verhindern. Zu dem ist es mittlerweile zu Resistenzen gegen die bekannten Medikamente gekommen.

Die vorliegende Erfindung eröffnet die Möglichkeit, mit einem besonders wirksamen Medikament/Nahrungsergänzungsmittel eine effektive Behandlung von Herpes genitalis sowie von Herpes labialis zu erzielen.

Grundsätzlich sind auch Abweichungen von dem oben näher beschriebenen Ausführungsbeispiel der Erfindung umfasst. Bei dem Lösungsmittel muss es sich nicht um ein alkoholisches Lösungsmittel handeln. Grundsätzlich kommen auch andere geeignete Lösungsmittel in Betracht. Wie oben ausgeführt, ist die Erfindung weiter nicht auf die Applikation als flüssiges Medikament beschränkt. Ebenfalls ist es denkbar, das Medikament in fester Form (Tablette, Pulver, Globuli) oder als Salbe bereitzustellen.

## Patentansprüche

1. Arzneimittel und/oder Nahrungsergänzungsmittel mit antiviralen Wirkungen, insbesondere zur präventiven und/oder therapeutischen Behandlung von Herpes labialis und/oder Herpes genitalis, enthaltend Nisylen und Cepa und Euphrasia und Belladonna und Mercurius solubilis.

2. Verwendung von Nisylen und Cepa und Euphrasia und Belladonna und Mercurius solubilis zur Herstellung eines Arzneimittels und/oder Nahrungsergänzungsmittels mit antiviralen Wirkungen, insbesondere zur präventiven und/oder therapeutischen Behandlung von Herpes labialis und/oder Herpes genitalis.

3. Arzneimittel und/oder Nahrungsergänzungsmittel oder Verwendung nach einem der Ansprüche 1 oder 2, **dadurch gekennzeichnet, dass** das Arzneimittel Nisylen als Hauptbestandteil und die weiteren Bestandteile als Nebenbestandteile enthält.

4. Arzneimittel und/oder Nahrungsergänzungsmittel oder Verwendung nach einem der Ansprüche 1 oder 2, **dadurch gekennzeichnet, dass** das Arzneimittel Cepa als Hauptbestandteil und die weiteren Bestandteile als Nebenbestandteile enthält.

5. Arzneimittel und/oder Nahrungsergänzungsmittel oder Verwendung nach einem der Ansprüche 1 oder 2, **dadurch gekennzeichnet, dass** das Arzneimittel Euphrasia als Hauptbestandteil und die weiteren Bestandteile als Nebenbestandteile enthält.

6. Arzneimittel und/oder Nahrungsergänzungsmittel oder Verwendung nach einem der Ansprüche 1 oder 2, **dadurch gekennzeichnet, dass** das Arzneimittel Belladonna als Hauptbestandteil und die weiteren Bestandteile als Nebenbestandteile enthält.

7. Arzneimittel und/oder Nahrungsergänzungsmittel oder Verwendung nach einem der Ansprüche 1 oder 2, **dadurch gekennzeichnet, dass** das Arzneimittel Mercurius solubilis als Hauptbestandteil und die weiteren Bestandteile als Nebenbestandteile enthält.

8. Arzneimittel und/oder Nahrungsergänzungsmittel oder Verwendung nach einem der vorhergehenden Ansprüche, **dadurch gekennzeichnet, dass** die Bestandteile Cepa, Euphrasia und Mercurius solubilis in der Potenz D12 und Belladonna in der Potenz D15 vorliegen.

9. Arzneimittel und/oder Nahrungsergänzungsmittel oder Verwendung nach einem der vorhergehenden Ansprüche, **dadurch gekennzeichnet, dass** das Arzneimittel Nisylen, Cepa, Euphrasia, Belladonna und Mercurius solubilis enthält, wobei Nisylen in dem Arzneimittel in größerer Menge vorliegt als die weiteren Bestandteile.

10. Arzneimittel und/oder Nahrungsergänzungsmittel oder Verwendung nach einem der vorhergehenden Ansprüche, **dadurch gekennzeichnet, dass** der Bestandteil Nisylen in zwei- bis vierfacher, vorzugsweise in dreifacher Menge vorliegt, wie jeder der anderen Bestandteile.

11. Arzneimittel und/oder Nahrungsergänzungsmittel oder Verwendung nach einem der vorhergehenden Ansprüche, **dadurch gekennzeichnet, dass** das Arzneimittel Nisylen in einem Gewicht oder in einem Volumen von 4,0 bis 4,5 g oder ml, vorzugsweise in einem Gewicht oder einem Volumen von 4,286 g oder ml, und Cepa, Euphrasia, Belladonna und Mercurius solubilis jeweils in einem Gewicht oder in einem Volumen von 1,2 bis 1,6 g oder ml, vorzugsweise in einem Gewicht oder in einem Volumen von 1,429 g oder ml je 10 ml Lösung enthält.

12. Arzneimittel und/oder Nahrungsergänzungsmittel oder Verwendung nach einem der vorhergehenden Ansprüche, **dadurch gekennzeichnet, dass** das Arzneimittel in flüssiger Form vorliegt.

13. Arzneimittel und/oder Nahrungsergänzungsmittel oder Verwendung nach Anspruch 12, **dadurch gekennzeichnet, dass** es sich bei dem Arzneimittel um eine alkoholische Lösung handelt.

14. Arzneimittel und/oder Nahrungsergänzungsmittel oder Verwendung nach einem der Ansprüche 1 bis 11, **dadurch gekennzeichnet, dass** das Arzneimittel in fester oder pastöser Form vorliegt.

15. Arzneimittel und/oder Nahrungsergänzungsmittel oder Verwendung nach einem der vorhergehenden Ansprüche, **dadurch gekennzeichnet, dass** der Bestandteil Nisylen folgende Bestandteile enthält: Aconitum napellus, Gelsemium sempervirens, Cephaelis ipecacuanha, Phosphorus, Bryonia und Eupatorium perfoliatum.

16. Arzneimittel und/oder Nahrungsergänzungsmittel oder Verwendung nach Anspruch 15, **dadurch gekennzeichnet, dass** pro 1 g Nisylen die Bestandteile in folgenden Mengen enthalten sind: 100 mg Aconitum napellus D3, 100 mg Gelsemium sempervirens D3, 100 mg Cephaelis ipecacuanha D3, 100 mg Phosphorus D5, 100 mg Bryonia D2 und 100 mg Eupatorium perfoliatum D1.

## Claims

1. Medicinal and/or food supplement product having antiviral effects, in particular for preventive and/or therapeutic treatment of herpes labialis and/or herpes genitalis, containing Nisylen and Cepa and Euphrasia and Belladonna and Mercurius solubilis.

2. Use of Nisylen and Cepa and Euphrasia and Belladonna and Mercurius solubilis in the manufacture of a medicinal and/or food supplement product having antiviral effects, in particular for preventive and/or therapeutic treatment of herpes labialis and/or herpes genitalis.

3. Medicinal and/or food supplement product or use according to either of Claims 1 and 2, **characterized in that** the medicinal product contains Nisylen as main constituent and the further constituents as secondary constituents.

4. Medicinal and/or food supplement product or use according to either of Claims 1 and 2, **characterized in that** the medicinal product contains Cepa as main constituent and the further constituents as secondary constituents.

5. Medicinal and/or food supplement product or use according to either of Claims 1 and 2, **characterized in that** the medicinal product contains Euphrasia as main constituent and the further constituents as secondary constituents.

6. Medicinal and/or food supplement product or use according to either of Claims 1 and 2, **characterized in that** the medicinal product contains Belladonna as main constituent and the further constituents as secondary constituents.

7. Medicinal and/or food supplement product or use according to either of Claims 1 and 2, **characterized in that** the medicinal product contains Mercurius solubilis as main constituent and the further constituents as secondary constituents.

8. Medicinal and/or food supplement product or use according to any preceding claim, **characterized in that** the constituents Cepa, Euphrasia and Mercurius solubilis are present in the potentiation D12 and Belladonna is present in the potentiation D15.

9. Medicinal and/or food supplement product or use according to any preceding claim, **characterized in that** the medicinal product contains Nisylen, Cepa, Euphrasia, Belladonna and Mercurius solubilis and Nisylen is present in the medicinal product in a larger amount than the other constituents.

10. Medicinal and/or food supplement product or use according to any preceding claim, **characterized in that** the constituent Nisylen is present in two to four times the amount, preferably in three times the amount, of any of the other constituents.

11. Medicinal and/or food supplement product or use according to any preceding claim, **characterized in that** the medicinal product contains Nisylen in a weight or volume of 4.0 to 4.5 g or ml, preferably in a weight or volume of 4.286 g or ml, and Cepa, Euphrasia, Belladonna and Mercurius solubilis each in a weight or volume of 1.2 to 1.6 g or ml, preferably in a weight or volume of 1.429 g or ml per 10 ml of solution.

12. Medicinal and/or food supplement product or use according to any preceding claim, **characterized in that** the medicinal product is present in liquid form.

13. Medicinal and/or food supplement product or use according to Claim 12, **characterized in that** the medicinal product comprises an alcoholic solution.

14. Medicinal and/or food supplement product or use according to any one of Claims 1 to 11, **characterized in that** the medicinal product is present in solid or pasty form.

15. Medicinal and/or food supplement product or use according to any preceding claim, **characterized in that** the constituent Nisylen contains the following constituents: Aconitum napellus, Gelsemium sempervirens, Cephaelis ipecacuanha, Phosphorus, Bryonia and Eupatorium perfoliatum.

16. Medicinal and/or food supplement product or use according to Claim 15, **characterized in that** per 1 g of Nisylen the constituents are included in the following amounts: 100 mg of Aconitum napellus D3, 100 mg of Gelsemium sempervirens D3, 100 mg of Cephaelis ipecacuanha D3, 100 mg of Phosphorus D5, 100 mg of Bryonia D2 and 100 mg of Eupatorium perfoliatum D1.

## Revendications

1. Composition pharmaceutique et/ou complément alimentaire avec des effets antiviraux, en particulier pour le traitement préventif ou thérapeutique de l'herpès labial et/ou de l'herpès génital, contenant les produits nisyline, cepa, euphrasia, belladonna et mercurius solubilis.

2. Utilisation de nisyline, cepa, euphrasia, belladonna et mercurius solubilis à la préparation d'une composition pharmaceutique et/ou d'un complément alimentaire avec des effets antiviraux, en particulier pour le traitement préventif ou thérapeutique de l'herpès labial et/ou de l'herpès génital.

3. Composition pharmaceutique et/ou complément alimentaire ou utilisation selon l'une quelconque des revendications 1 ou 2, **caractérisés en ce que** la composition pharmaceutique contient de la nisyline comme composant principal et les autres composants comme composants secondaires.

4. Composition pharmaceutique et/ou complément alimentaire ou utilisation selon l'une quelconque des revendications 1 ou 2, **caractérisés en ce que** la composition pharmaceutique contient du cepa comme composant principal et les autres composants comme composants secondaires.

5. Composition pharmaceutique et/ou complément alimentaire ou utilisation selon l'une quelconque des revendications 1 ou 2, **caractérisés en ce que** la composition pharmaceutique contient de l'euphrasia comme composant principal et les autres composants comme composants secondaires.

6. Composition pharmaceutique et/ou complément alimentaire ou utilisation selon l'une quelconque des revendications 1 ou 2, **caractérisés en ce que** la composition pharmaceutique contient de la belladonna comme composant principal et les autres composants comme composants secondaires.

7. Composition pharmaceutique et/ou complément alimentaire ou utilisation selon l'une quelconque des revendications 1 ou 2, **caractérisés en ce que** la composition pharmaceutique contient du mercurius solubilis comme composant principal et les autres composants comme composants secondaires.

8. Composition pharmaceutique et/ou complément alimentaire ou utilisation selon l'une quelconque des revendications précédentes, **caractérisés en ce que** les composants cepa, euphrasia et mercurius solubilis sont présents à la puissance D12 et la belladonna à la puissance D15.

9. Composition pharmaceutique et/ou complément alimentaire ou utilisation selon l'une quelconque des revendications précédentes, **caractérisés en ce que** la composition pharmaceutique contient de la nisyline, du cepa, dé l'euphrasia, de la belladonna et du mercurius solubilis, sachant que la nisyline est présente dans la composition pharmaceutique en plus grande quantité que les autres composants.

10. Composition pharmaceutique et/ou complément alimentaire ou utilisation selon l'une quelconque des revendications précédentes, **caractérisés en ce que** le composant nisyline est présent en quantité double à quadruple, de préférence triple de celle de chacun des autres composants.

11. Composition pharmaceutique et/ou complément alimentaire ou utilisation selon l'une quelconque des revendications précédentes, **caractérisés en ce que** la composition pharmaceutique contient la nisyline en un poids ou en un volume de 4,0 à 4,5 g ou ml, de préférence en un poids ou un volume de 4,286 g ou ml, et le cepa, l'euphrasia, la belladonna et le mercurius solubilis chacun en un poids ou un volume de 1,2 à 1,6 g ou ml, de préférence en un poids ou un volume de 1,429 g ou ml par 10 ml de solution.

12. Composition pharmaceutique et/ou complément alimentaire ou utilisation selon l'une quelconque des revendications précédentes, **caractérisés en ce que** la composition pharmaceutique est présente sous forme liquide.

13. Composition pharmaceutique et/ou complément alimentaire ou utilisation selon la revendication 12, **caractérisés en ce qu'**il s'agit pour la composition pharmaceutique d'une solution alcoolique.

14. Composition pharmaceutique et/ou complément alimentaire ou utilisation selon l'une quelconque des revendications 1 à 11, **caractérisés en ce que** la composition pharmaceutique est présente sous forme solide ou pâteuse.

15. Composition pharmaceutique et/ou complément alimentaire ou utilisation selon l'une quelconque des revendications précédentes, **caractérisés en ce que** le composant nisyline contient les composants: aconitum napellus, gelsemium sempervirens, cepaelis ipecacuanha, phosphorus, byronia et eupatorium perfoliatum.

16. Composition pharmaceutique et/ou complément alimentaire ou utilisation selon la revendication 15, **caractérisés en ce que** par 1 g de nisyline, les composants sont présents dans les quantités suivantes: 100 mg de aconitum napellus D3, 100 mg de gelsemium sempervirens D3, 100 mg de cepaelis ipecacuanha D3, 100 mg de phosphorus D5, 100 mg de byronia D2 et 100 mg de eupatorium perfoliatum D1.
